# EUROPÄISCHE PATENTSCHRIFT

(11) **EP 2 258 256 B1**
(45) Veröffentlichungstag und Bekanntmachung des Hinweises auf die Patenterteilung: **13.03.2013**
(21) Anmeldenummer: 10164555.4
(22) Anmeldetag: 01.06.2010
(51) Int. Cl.: A61B 3/103, A61B 3/107

(54) **Verfahren zur Bestimmung einer Kontaktlinse**
Method for determining a contact lens
Procédé de détermination d'une lentille de contact

(30) Priorität: 02.06.2009 DE 102009023462
(43) Veröffentlichungstag der Anmeldung: 08.12.2010
(73) Patentinhaber: Oculus Optikgeräte GmbH, 35582 Wetzlar (DE)
(72) Erfinder: Steinmüller, Andreas, 35435, Wettenberg (DE)
(74) Vertreter: Tappe, Hartmut

(56) Entgegenhaltungen:
- EP-A1- 1 844 704
- US-A- 3 108 523
- US-A- 4 878 750
- US-A- 5 414 478
- US-A1- 2004 246 440
- US-B1- 6 379 008

## Beschreibung

Die Erfindung betrifft ein Verfahren zur Bestimmung und Auswahl einer Kontaktlinse mit einem ophthalmologischen Gerät zur Augenuntersuchung, gebildet aus einem Keratometer und einem Autorefraktometer sowie Mitteln zur Datenverarbeitung, wobei eine Brechkraft eines Auges und eine Topographie einer Hornhaut ermittelt wird, wobei eine Refraktion eines zu untersuchenden Auges beschreibende Refraktionsdaten gewonnen werden, wobei eine Topographie einer Hornhaut des Auges beschreibende Topographiedaten gewonnen werden. Weiter betrifft die Erfindung ein ophthalmologisches Gerät zur Durchführung des Verfahrens.

Zur Bestimmung und Auswahl einer Kontaktlinse, insbesondere einer weichen Kontaktlinse, ist es notwendig, eine Brechkraft eines Auges, dem die Kontaktlinse angepasst werden soll, und eine Topographie einer Hornhaut des Auges zu ermitteln. Ausgehend von diesen Daten muss dann eine geeignete Kontaktlinse eines Herstellers ausgewählt werden. Dazu sind relativ umfangreiche Berechnungen notwendig, die sich je nach Hersteller unterscheiden können, da die Hersteller regelmäßig voneinander abweichende Anpassungsregeln vorsehen. Weiter muss ein Durchmesser einer Kontaktlinse auf einen Durchmesser der Hornhaut abgestimmt sein. Da die Hersteller eine Reihe von Kontaktlinsen zur Verfügung stellen, die in ihren technischen Daten beziehungsweise Abmessungen sich so unterscheiden, dass nicht alle ermittelten Messwerte abgedeckt werden können, bedarf es eines iterativen Vorgangs zur Auswahl einer Kontaktlinse. Das heißt nach einer Berechnung und einer Vorauswahl von geeigneten Kontaktlinsen durch eine untersuchende Person wird einem Patienten eine Reihe von Kontaktlinsen angepasst, wobei eine geeignete Kontaktlinse aufgrund eines subjektiven Seheindrucks des Patienten ausgewählt wird. Nachteilig ist hier insbesondere, dass die untersuchende Person relativ gut in der Berechnung beziehungsweise Auswahl von Kontaktlinsen ausgebildet sein muss, und dass der Vorgang der Berechnung und Auswahl der geeigneten Kontaktlinse vergleichsweise viel Zeit in Anspruch nimmt.

Ein Gerät und ein Verfahren zur Auswahl einer Kontaktlinse ist bereits aus dem Dokument US 4 878 750 bekannt.

Der vorliegenden Erfindung liegt daher die Aufgabe zugrunde, ein Verfahren bzw. ein ophthalmologisches Gerät vorzuschlagen, welches eine Berechnung und Anpassung einer Kontaktlinse vereinfacht.

Diese Aufgabe wird durch ein Verfahren mit den Merkmalen des Anspruchs 1 und ein ophthalmologisches Gerät mit den Merkmalen des Anspruchs 15 gelöst.

Bei dem erfindungsgemäßen Verfahren zur Bestimmung und Auswahl einer Kontaktlinse mit einem ophthalmologischen Gerät zur Augenuntersuchung, welches aus einem Keratometer und einem Autorefraktometer sowie Mitteln zur Datenverarbeitung gebildet ist, wird eine Brechkraft eines Auges und eine Topographie einer Hornhaut ermittelt, wobei eine Refraktion eines zu untersuchenden Auges beschreibende Refraktionsdaten gewonnen werden, wobei eine Topographie einer Hornhaut des Auges beschreibende Topographiedaten gewonnen werden, wobei mittels der gewonnen Refraktions- und Topographiedaten Kontaktlinsendaten berechnet werden, und aus einer Datenbank des Gerätes eine Kontaktlinse ausgewählt wird.

Dadurch ist der Vorteil erzielbar, dass das ophthalmologische Gerät die Berechnung der Kontaktlinse durchführt und aus einer Datenbank mit Kontaktlinsendaten die geeignete Kontaktlinse auswählt. Da so die geeignete Kontaktlinse relativ genau ermittelt werden kann, sind weder umständliche Berechnungen durch die untersuchende Person noch langwierige Testreihen mit verschiedenen Kontaktlinsen an einem Patienten notwendig.

Vorteilhaft kann ein Vergleich von berechneten Kontaktlinsendaten mit in der Datenbank enthaltenen Kontaktlinsendaten erfolgen. So kann aus den objektiven Messdaten eine notwendige Soll-Kontaktlinse berechnet werden, wobei mit diesen Daten aus der im Gerät vorhandenen Datenbank eine Ist-Kontaktlinse ausgewählt wird, die im Hinblick auf die Soll-Kontaktlinse am geeignetsten erscheint. Ein Kriterium für eine Eignung kann eine weitestgehende Übereinstimmung der berechneten Kontaktlinsendaten mit den in der Datenbank enthaltenen Kontaktlinsendaten sein.

In einer Ausführungsform des Verfahrens kann eine Ausgabe der Messdaten, der berechneten Kontaktlinsendaten und der Kontaktlinsendaten der ausgewählten Kontaktlinse erfolgen. Dies kann beispielsweise auch in Form einer Anzeige auf einem Bildschirm oder in Form eines Ausdrucks, welcher einer Patientenakte hinzugefügt werden kann, erfolgen. Auch kann dann eine untersuchende Person eine vom Gerät durchgeführte Auswahl einer Plausibilitätsprüfung unterziehen.

Weiter kann es vorgesehen sein, mehrere Berechnungen in verschiedenen Berechnungsmodi durchzuführen. Das heißt die gewonnenen Daten können mehreren Berechnungen unterworfen werden, welche jeweils auf verschiedenen Berechnungsmodi basieren. Die hierzu verwendeten Berechnungsmodi können an Berechnungsmodi beziehungsweise Kontaktlinsenanpassungsregeln von Kontaktlinsenherstellern angepasst oder angenähert sein. Somit können bestimmte herstellerspezifische Abweichungen bei der Angabe von Kontaktlinsendaten bereits bei der Berechnung berücksichtigt werden.

Insbesondere können Anpassungsregeln für Kontaktlinsen unterschiedlichster Hersteller berücksichtigt werden.

Weiter können auch bei einer Auswahl einer Kontaktlinse jeweils Kontaktlinsenanpassungsregeln berücksichtig werden. Das heißt für nachfolgende Berechnungen werden gegebenenfalls ergänzend verschiedene Kontaktlinsenanpassungsregeln von Kontaktlinsenherstellern für eine Auswahl einer geeigneten Kontaktlinse herangezogen.

Vorteilhaft können Messdaten mit einer einzelnen Messung des Gerätes gewonnen werden. Mit einer Messung können beispielsweise alle notwendigen physiologischen Daten des betreffenden Auges, wie Sphäre, Zylinder, Achse, Brechkraft der Hornhaut beziehungsweise des gesamten Auges, ermittelt werden. Es ist daher nicht mehr notwendig, zwei aufeinanderfolgende Messungen, mit einem Autorefraktometer und einem Topographiesystem, an einem Auge durchzuführen. Dadurch wird ein Auswahlverfahren weiter beschleunigt, und eine eventuelle Verfälschung von Messergebnissen durch eine Änderung von Messbedingungen kann ausgeschlossen werden.

Auch kann vorgesehen sein, dass Messdaten von Hornhautzentralradien einer Hornhaut gewonnen werden. Diese Messung kann vorzugsweise mit dem Keratometer erfolgen.

Es ist vorgesehen, dass Messdaten eines Hornhautdurchmessers einer Hornhaut gewonnen werden. Eine sogenannte "white to white" Messung umfasst eine Messung des Hornhautdurchmessers, welcher im wesentlichen von einem Außendurchmesser einer Iris bestimmt wird. Die Messung des Hornhautdurchmessers ist insbesondere bedeutend für eine Auswahl weicher Kontaktlinsen beziehungsweise deren Durchmesser sowie für eine Prüfung einer Kontaktlinsenauflage auf der Hornhaut.

Darüber hinaus können über einen Bereich des Hornhautdurchmessers hinausgehende Messdaten der Hornhaut gewonnen werden. Das heißt es können nicht nur Zentralradien der Hornhaut im Bereich der Iris ermittelt werden, sondern auch Krümmungsradien oder eine Hornhauttopographie in den über die Iris hinaus gehenden Hornhautbereichen. Demnach kann das Keratometer so ausgebildet sein, dass die gesamte Hornhaut gemessen wird beziehungsweise entsprechende Topographiedaten gewonnen werden.

Das Messverfahren kann weiter dazu verwendet werden, einen Keratokonus einer Hornhaut zu bestimmen, und Messdaten desselben zu gewinnen. Da ein Keratokonus regelmäßig nicht zentral auf der Hornhaut ausgebildet ist, ist dessen Bestimmung mittels eines Topographiesystems, welches alleine zur Gewinnung von Messdaten zur Auswahl einer Kontaktlinse ausgebildet ist, nicht oder nur eingeschränkt möglich. Beispielsweise kann durch die Verwendung eines Keratometers mit einer einzelnen, ringförmigen Beleuchtungsquelle bereits ein auf einer Hornhaut ausgebildeter Keratokonus festgestellt werden. Die Bestimmung eines Keratokonus kann unter anderem zu einer Auswahl von beispielsweise harten Kontaktlinsen führen, da diese den Keratokonus vorteilhaft abflachen können.

Mittels des ophthalmoligischen Gerätes kann auch ein Schnittbild eines Auges gewonnen werden, wobei die sich aus dem Schnittbild ergebenden Bilddaten bei der Berechnung der Kontaktlinsendaten verwendet werden können. Mit Hilfe des Schnittbildes können unter anderem auch eine Hornhautdicke sowie Krümmungsradien einer Hornhaut bestimmt werden. Weiter eignet sich ein Schnittbild, welches mittels einer Spaltbeleuchtung und einer Kamera in einer Scheimpfluganordnung erzeugt werden kann, besonders gut zur Messung eines Keratokonus und einer in diesem Bereich verringerten Hornhautdicke.

Vorteilhaft kann die Datenbank Kontaktlinsendaten und Kontaktlinsenanpassungsregeln verschiedener Kontaktlinsenhersteller umfassen. So wird es möglich eine für den Patienten besonders gut passende Kontaktlinse auszuwählen.

Auch können bei einer Auswahl Materialdaten von Kontaktlinsen berücksichtigt werden beziehungsweise in der Datenbank enthalten sein. Neben den vorgenannten Daten können noch weitere, weiche Kontaktlinsen betreffende Daten, wie beispielsweise eine Luftdurchlässigkeit des Kontaktlinsenmaterials und sonstige Materialdaten berücksichtigt werden.

Besonders vorteilhaft ist es, wenn Informationen zur Verfügbarkeit der vorgeschlagenen Kontaktlinse ausgegeben werden. So sind Kontaktlinsen in verschiedenen Größen- und/oder Sehstärkeabstufungen erhältlich. In die Auswahl einer Kontaktlinse können weiter eventuelle Angaben über Lagerhaltung und Lieferzeiten der jeweiligen Kontaktlinsen mit einfließen. So ist es möglich, neben der aus physiologischen Gesichtspunkten geeignetsten Kontaktlinse auch eine Auswahlentscheidung unter Einbeziehung von deren Verfügbarkeit treffen.

Sinngemäß ist es vorteilhaft, wenn die Datenbank mit einer geräteexternen Datenbank abgeglichen werden kann. Die Daten können beispielsweise auf einem PC vorkonfiguriert werden und an das ophthalmologische Gerät übertragen werden. So ist es möglich, stets das aktuelle Angebot an Kontaktlinsen zu berücksichtigen. Eine Übertragung kann beispielsweise per WLAN erfolgen. Auch ist es denkbar, dass eine direkte Verbindung zu Herstellerdatenbanken von dem Gerät selbst über das Internet aufgebaut wird.

Bei dem erfindungsgemäßen ophthalmologischen Gerät zur Durchführung des erfindungsgemäßen Verfahrens ist vorgesehen, dass das Keratometer, das Autorefraktometer und die Mittel zur Datenverarbeitung in einem gemeinsamen Gehäuse des ophthalmologischen Gerätes angeordnet sind. Somit ist es nicht mehr notwendig, mehrere Geräte miteinander zu verbinden.

Weitere vorteilhafte Ausführungsformen eines ophthalmologischen Gerätes ergeben sich aus den Merkmalsbeschreibungen der auf den Verfahrensanspruch 1 rückbezogenen Unteransprüche.

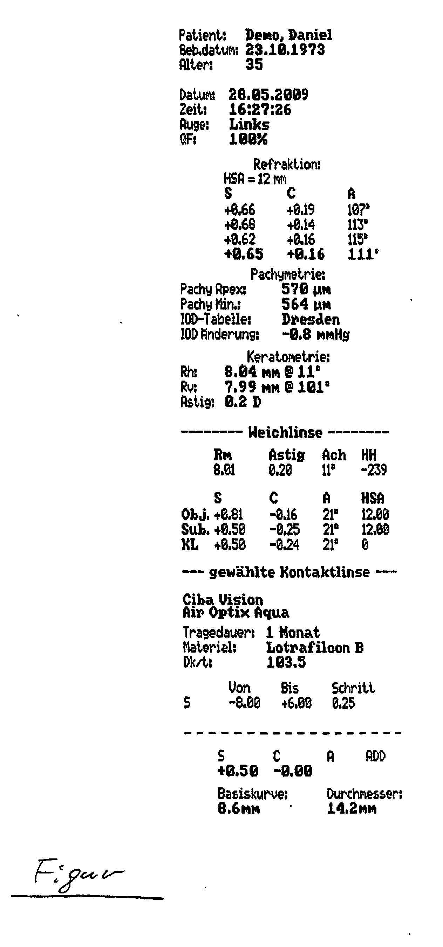

Im Folgenden wird die Erfindung unter Bezugnahme auf die beigefügte Figur näher erläutert.

Eine Darstellung der ermittelten Messwerte und der ausgewählten Kontaktlinse kann zum Beispiel in Art eines Ausdrucks, wie in der Figur dargestellt, von dem Gerät ausgegeben werden. Wie aus dem Beispiel ersichtlich ist, werden neben den objektiven Messdaten die Daten einer notwendigen Kontaktlinse dargestellt, anhand derer eine Kontaktlinse aus einer Datenbank ausgewählt wurde. Zu der ausgewählten Kontaktlinse werden weitere Informationen zu verfügbaren Abstufungen der Brechkraft und zu dem berechneten Kontaktlinsendurchmesser ausgegeben.

## Patentansprüche

1. Verfahren zur Bestimmung und Auswahl einer Kontaktlinse mit einem ophthalmologischen Gerät zur Augenuntersuchung, gebildet aus einem Keratometer und einem Autorefraktometer sowie Mitteln zur Datenverarbeitung, wobei eine Brechkraft eines Auges und eine Topographie einer Hornhaut ermittelt wird, wobei eine Refraktion eines zu untersuchenden Auges beschreibende Refraktionsdaten gewonnen werden, wobei eine Topographie einer Hornhaut des Auges beschreibende Topographiedaten gewonnen werden, wobei mittels der gewonnenen Refraktions- und Topographiedaten Kontaktlinsendaten berechnet werden, und aus einer Datenbank des Gerätes durch das Gerät eine Kontaktlinse ausgewählt wird,
**dadurch gekennzeichnet,**
**dass** Messdaten eines Hornhautdurchmessers der Hornhaut mittels des Keratometers gewonnen und zur Auswahl der Kontaktlinse herangezogen werden.

2. Verfahren nach Anspruch 1,
**dadurch gekennzeichnet,**
**dass** ein Vergleich von berechneten Kontaktlinsendaten mit in der Datenbank enthaltenen Kontaktlinsendaten erfolgt.

3. Verfahren nach Anspruch 1 oder 2,
**dadurch gekennzeichnet,**
**dass** eine Ausgabe der Messdaten, der berechneten Kontaktlinsendaten und der Kontaktlinsendaten der ausgewählten Kontaktlinse erfolgt.

4. Verfahren nach einem der vorangehenden Ansprüche,
**dadurch gekennzeichnet,**
**dass** mehrere Berechnungen in verschiedenen Berechnungsmodi durchgeführt werden.

5. Verfahren nach einem der vorangehenden Ansprüche,
**dadurch gekennzeichnet,**
**dass** bei einer Auswahl jeweils Kontaktlinsenanpassungsregeln berücksichtigt werden.

6. Verfahren nach einem der vorangehenden Ansprüche,
**dadurch gekennzeichnet,**
**dass** Messdaten mit einer einzelnen Messung des Gerätes gewonnen werden.

7. Verfahren nach einem der vorangehenden Ansprüche,
**dadurch gekennzeichnet,**
**dass** Messdaten von Hornhautzentralradien der Hornhaut gewonnen werden.

8. Verfahren nach Anspruch 7,
**dadurch gekennzeichnet,**
**dass** über einen Bereich des Hornhautdurchmessers hinausgehende Messdaten der Hornhaut gewonnen werden.

9. Verfahren nach einem der vorangehenden Ansprüche,
**dadurch gekennzeichnet,**
**dass** ein Keratokonus der Hornhaut bestimmt wird, und Messdaten desselben gewonnen werden.

10. Verfahren nach einem der vorangehenden Ansprüche,
**dadurch gekennzeichnet,**
**dass** ein Schnittbild eines Auges gewonnen wird, und dass entsprechende Bilddaten bei der Berechnung verwendet werden.

11. Verfahren nach einem der vorangehenden Ansprüche,
**dadurch gekennzeichnet,**
**dass** die Datenbank Kontaktlinsendaten und Kontaktlinsenanpassungsregeln verschiedener Kontaktlinsenhersteller umfasst.

12. Verfahren nach einem der vorangehenden Ansprüche,
**dadurch gekennzeichnet,**
**dass** Materialdaten von Kontaktlinsen berücksichtigt werden.

13. Verfahren nach einem der vorangehenden Ansprüche,
**dadurch gekennzeichnet,**
**dass** Informationen zur Verfügbarkeit der vorgeschlagenen Kontaktlinse ausgegeben werden.

14. Verfahren nach einem der vorangehenden Ansprüche,
**dadurch gekennzeichnet,**
**dass** die Datenbank mit einer geräteexternen Datenbank abgeglichen wird.

15. Ophthalmologisches Gerät zur Durchführung des Verfahrens nach einem der vorangehenden Ansprüche,
**dadurch gekennzeichnet,**
**dass** das Keratometer, das Autorefraktometer und die Mittel zur Datenverarbeitung in einem gemeinsamen Gehäuse des ophthalmologischen Gerätes angeordnet sind.

## Claims

1. A method of determining and selecting a contact lens with an ophthalmological device for examining the eyes comprising a keratometer and an autorefractometer as well as means of data processing, wherein a refractive power of an eye and a topography of a cornea are determined, wherein refraction data describing a refraction of an eye to be examined are obtained, wherein topography data describing the topography of the cornea of the eye are obtained, wherein contact lens data are calculated on the basis of the obtained refraction and topography data and a contact lens is selected from a database of the device by the device,
**characterised in that**
measuring data of a corneal diameter of the cornea are obtained by means of the keratometer and are used for selecting the contact lens.

2. The method according to claim 1,
**characterised in that**
a comparison of the calculated contact lens data with the contact lens data contained in the date base is carried out.

3. The method according to claim 1 or 2,
**characterised in that**
the measuring data, the calculated contact lens data and the contact lens data of the selected contact lens are output.

4. The method according to any one of the preceding claims,
**characterised in that**
several calculations in various calculation modes are carried out.

5. The method according to any one of the preceding claims,
**characterised in that**
contact lens adaptation rules are taken into account in each selection.

6. The method according to any one of the preceding claims,
**characterised in that**
measuring data are obtained with a single measurement by the device.

7. The method according to any one of the preceding claims,
**characterised in that**
measuring data of corneal central radii of the cornea are obtained.

8. The method according to claim 7,
**characterised in that**
measuring data of the cornea going beyond a range of the corneal diameter are obtained.

9. The method according to any one of the preceding claims,
**characterised in that**
a keratoconus of the cornea is determined and measuring data thereof are obtained.

10. The method according to any one of the preceding claims,
**characterised in that**
a cross-section of an eye is obtained and the corresponding image data are used in the calculation.

11. The method according to any one of the preceding claims,
**characterised in that**
the database comprises contact lens data and contact lens adaptation rules of various contact lens manufacturers.

12. The method according to any one of the preceding claims,
**characterised in that**
material data of contact lenses are taken into account.

13. The method according to any one of the preceding claims,
**characterised in that**
information relating to the availability of the proposed contact lens is issued.

14. The method according to any one of the preceding claims,
**characterised in that**
the database is coordinated with a device-external database.

15. An ophthalmological device for implementing the method according to any one of the preceding claims,
**characterised in that**
the keratometer, the autorefractometer and the means of data processing are arranged in a joint housing of the ophthalmological device.

## Revendications

1. Procédé de détermination et de sélection d'une lentille de contact avec un appareil ophtalmologique pour examiner les yeux, formé par un kératomètre et un auto-réfractomètre ainsi que par des moyens de traitement des données, dans lequel un pouvoir de réfraction d'un oeil et une topographie d'une cornée sont déterminés, dans lequel des données réfractives sont obtenues qui décrivent une réfraction d'un oeil à examiner, dans lequel des données topographiques sont obtenues qui décrivent une topographie d'une cornée de l'oeil, dans lequel des données de lentilles de contact sont calculées à la base des données réfractives et topographiques obtenues et une lentille de contact est sélectionnée par l'appareil dans une base de données de l'appareil,
**caractérisé en ce que**
des données mesurées d'un diamètre cornéen de la cornée sont obtenues au moyen du kératomètre et sont utilisées pour sélectionner la lentille de contact.

2. Procédé selon la revendication 1,
**caractérisé en ce que**
les données de lentilles de contact calculées sont comparées avec les données de lentilles de contact contenues dans la base de données.

3. Procédé selon la revendication 1 ou 2,
**caractérisé en ce que**
les données mesurées, les données de lentilles de contact calculées et les données de lentilles de contact de la lentille de contact sélectionnée sont émises.

4. Procédé selon l'une des revendications précédentes,
**caractérisé en ce que**
plusieurs calculs sont effectués selon des modes de calcul différents.

5. Procédé selon l'une des revendications précédentes,
**caractérisé en ce que**
des règles d'adaptation de lentilles de contact sont pris en compte lors de chaque sélection.

6. Procédé selon l'une des revendications précédentes,
**caractérisé en ce que**
les données mesurées sont obtenues avec une seule mesure de l'appareil.

7. Procédé selon l'une des revendications précédentes,
**caractérisé en ce que**
des données mesurées sont obtenues des rayons cornéens centraux de la cornée.

8. Procédé selon la revendication 7,
**caractérisé en ce que**
des données mesurées de la cornée qui sont obtenues dépassent une zone du diamètre cornéen.

9. Procédé selon l'une des revendications précédentes,
**caractérisé en ce que**
un kératocône de la cornée est identifié et des données mesurées du même sont obtenues.

10. Procédé selon l'une des revendications précédentes,
**caractérisé en ce que**
une coupe transversale d'un oeil est obtenue et des données d'image correspondants sont utilisées lors du calcul.

11. Procédé selon l'une des revendications précédentes,
**caractérisé en ce que**
la base de données comprend des données de lentilles de contact et des règles d'adaptation de lentilles de contact de fabricants de lentilles de contact différents.

12. Procédé selon l'une des revendications précédentes,
**caractérisé en ce que**
des données de matériaux des lentilles de contact sont pris en compte.

13. Procédé selon l'une des revendications précédentes,
**caractérisé en ce que**
des informations sur la disponibilité de la lentille de contact proposée sont émises.

14. Procédé selon l'une des revendications précédentes,
**caractérisé en ce que**
la base de données est coordonnée avec une base de données externe.

15. Procédé selon l'une des revendications précédentes,
**caractérisé en ce que**
le kératomètre, l'auto-réfractomètre et les moyens de traitement des données sont arrangés dans un boîtier joint de l'appareil ophtalmologique.
